# EUROPEAN PATENT SPECIFICATION

(11) **EP 3 107 603 B1**
(45) Date of publication and mention of the grant of the patent: **28.03.2018**
(21) Application number: 14705178.3
(22) Date of filing: 18.02.2014
(51) Int. Cl.: A61M 5/28

(54) **DELIVERY SYSTEM FOR DELIVERING MEDICAL OR PHARMACEUTICAL COMPOUNDS**
AUSGABESYSTEM ZUR AUSGABE VON MEDIZINISCHEN ODER PHARMAZEUTISCHEN VERBINDUNGEN
SYSTEME D'ADMINISTRATION POUR L'ADMINISTRATION DE COMPOSES MEDICAUX OU PHARMACEUTIQUES

(43) Date of publication of application: 28.12.2016
(73) Proprietor: Skufca, Peter, 78333 Stockach (DE)
(72) Inventor: SKUFCA, Peter Maks, 1230 Domzale (SI)
(74) Representative: Winter, Brandl, Fürniss, Hübner, Röss, Kaiser, Polte - Partnerschaft mbB
(86) International application number: PCT/EP2014/053145
(87) International publication number: WO 2015/124172

(56) References cited:
- EP-A1- 2 535 073

## Description

The present invention refers to a delivery system for delivering medical or pharmaceutical compounds. The invention is defined by claim 1. A prior art delivery system for delivering medical or pharmaceutical compounds is described in EP 2 535 073 A1 and comprises a first container storing the compounds, a closure element accommodated within the first container, and an extraction unit adapted to extract the compounds automatically, once the closure element has been manually penetrated. In the known system, the first container is accommodated within a second container which serves, on the one hand, as holding means for holding an energy unit used to carry out the above mentioned automatic function and, on the other hand, as support means to assist the first container in maintaining the extraction unit in a well defined positional relationship with respect to the first container. That is, in order to put into effect the above mentioned automatic delivery function, a container-container-arrangement is provided that renders the overall structure complicate and accident-sensitive.
It is an object of the present invention to improve the reliability of the above known system by providing a delivery system for delivering medical or pharmaceutical compounds that dispenses with the above mentioned automatic function.
This object is solved by a delivery system having the features of claim 1. Advantageous embodiments and variations are defined in the dependent claims.
A delivery system for delivering medical or pharmaceutical compounds according to the present invention comprises (a) a container unit comprising (a-a) a container for storing the compounds, wherein the container has a circumferential wall, a first end closed by a container bottom and an open second end, and wherein the container unit has a longitudinal axis, and (a-b) a closure element accommodated within the container in a fluid-tight contact with the circumferential wall; and (b) an extraction unit comprising a supporting element supported at the closure element and a penetration and discharge means having a hollow needle adapted to penetrate the closure element. The penetration and discharge means is fitted to the supporting element so as be movable relatively to the supporting element along the longitudinal axis. According to the present invention, the extraction unit comprises a first link motion portion and a second link motion portion. The first link motion portion is provided at the penetration and discharge means, while the second link motion portion is provided at the supporting element. Further, one of the first link motion portion and the second link motion portion is provided with a projection and the respective other one is provided with a guiding groove, the projection and the guiding groove being engageable with each other to form a link motion adapted to make the penetration and discharge means move relatively to the supporting element in a predetermined way towards the container bottom thereby to make the hollow needle penetrate said closure element. That is, according to the present invention the extraction unit is, in structural and functional terms, divided into the penetration and discharge means comprising or forming the first link motion portion, and the supporting element comprising or forming the second link motion portion. As the supporting element is supported at the closure element, the penetration and discharge means is movable relatively to the closure element.

In terms of the support of the supporting element at the closure element, the supporting element may be coupled to the closure element in a form locking manner and/or force locking manner. A coupling in a form locking manner and/or force locking manner enables to transfer a longitudinal force from the penetration and discharge means to the supporting element and, thus, to the closure element. The supporting element may be stably coupled to the closure element, .e.g. by means of a press-fit or by means of bonding, or may be releasably coupled to the closure element, e.g. by means of press-fit or by means of screwing.

The word stems deliver, extract, and discharge each emphasize a specific perspective. That is, deliver refers to the inventive system in its entirety in the general meaning of dispense or give off or the more specific meaning of administer in case the subject the compounds are delivered to is an individual, extract refers to the extraction unit in order to focus on the process or operation of removing or taking out the compounds from the container disregarding the purpose of this process, i.e. irrespective of what is aimed to be achieved with the compounds, and discharge refers to the transport of the compounds from the container through the hollow needle to the outside of the (entire) system, i.e. objectively to the penetration and discharge means.

The medical or pharmaceutical compounds are liquids that are in contact with the container and the closure element. Therefore, the container together with the closure element may form or advantageously form a primary package for the compounds in conformity with the Guidelines on packaging for pharmaceutical Products, issued in the WHO Technical Report Series, No. 902, 2002, determining or defining that a primary packaging must protect the pharmaceutical or medical products against all adverse external influences that may affect its quality or potency such as, for example, light, moisture, oxygen, biological contamination or mechanical damage. In particular, such a primary packaging must not interact physically or chemically with the contended medical or pharmaceutical compounds in any way that would alter their quality. Specifically, a primary packaging must protect the contents from extraneous matter, from loss of the substance, and from efflorescence, deliquescence or evaporation under normal conditions of handling, shipment or storage.

The container and the closure element are parts of the container unit. That is, the container unit comprises at least these elements but may contain more than these elements.

Advantageously, the container is essentially or overall of a right-cylindrical shape, i.e. of the shape of a mathematical cylinder having its axis (the above mentioned longitudinal axis) perpendicular to its base. That is, the inventive container may be thought of as being made up of a tube or barrel (its circumferential or side wall) of undefined cross-section, having its longitudinal axis perpendicular to each of virtual planes closing its ends, where one of these ends (the first end) is, preferably integrally, connected with or firmly closed by the container bottom (i.e. the container bottom is not non-destructively removable) that may or may not completely lie within the respective virtual plane (it may, for example, be curved or bulged). Preferably, the cross-section is symmetric with respect to the longitudinal axis, so the longitudinal axis is an axis of symmetry.

Preferably, in order to enable handling and processing of the container in conventional filling facilities and with conventional technology used for filling and processing of standardized syringes, the container may meet, in terms of shape and dimensions, selected specifications of ISO 11040-4 standard of a prefilled syringe. Specifically, all cross-sections perpendicular to its longitudinal axis or axis of symmetry may be circles. Especially in this regard, the container may comprise or form a flange portion or form along its open second end.

The following three paragraphs give preferred dimensions according to various standards. Following the terminology used in these standards, the container without flange portion is called a barrel.

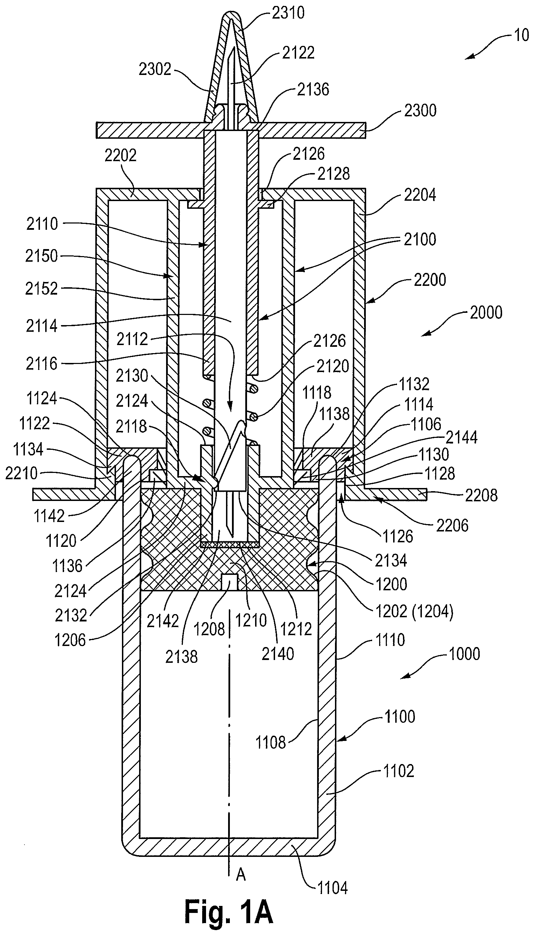

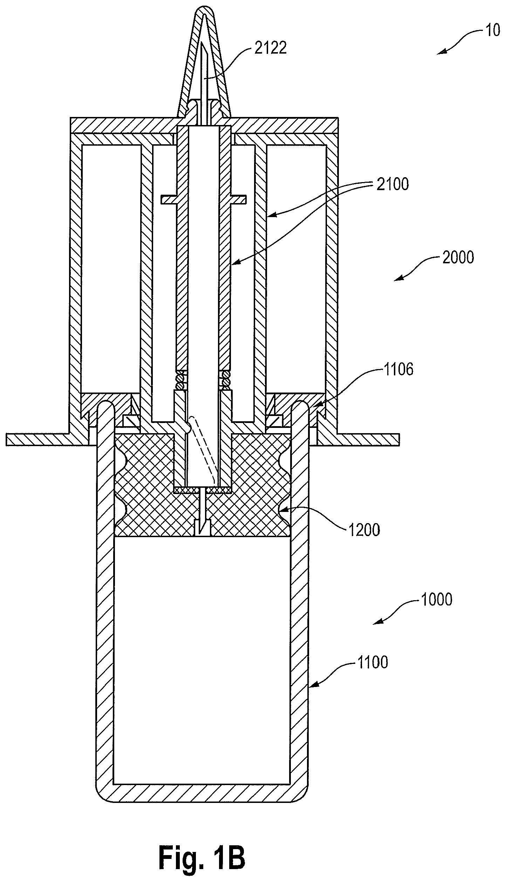

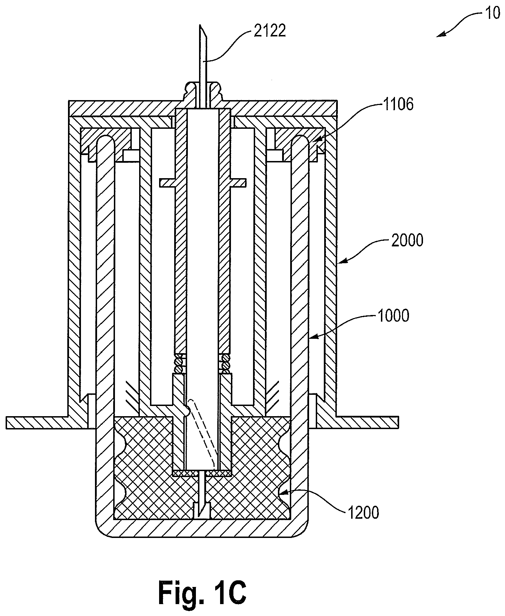

Preferably the cylindrical barrel complies, in terms of its inner diameter, outer diameter and wall thickness, with the relevant specifications of the above-mentioned ISO 11040-4 standard for a suitable specific standardized nominal volume. The specific standardized nominal volume may correspond to or may be close to, i.e. may slightly differ from, the predetermined filling volume of the container, i.e. is a suitable one of the various nominal volumes considered in the ISO 11040-4 standard. In particular, the cylindrical barrel may be formed to meet the barrel diameters d1 and d2 and the barrel wall thickness s1 as indicated in Figure 1 and Table 1 of the ISO 11040-4 standard for a specific standardized nominal volume of a standardized syringe. Depending on the predetermined filling volume, the length of the barrel may conform to the length l1 or total length I of a standardized syringe as indicated in Figure 1 and Table B.1 of ISO 11040-4 standard, or may vary within a range defined by the length l1 and the total length I as aforementioned, or may even be different from the specifications of the ISO 11040-4 standard. Generally, the length of the cylindrical barrel is defined and set so that the predetermined filling can be achieved with the inner diameter, outer diameter, and wall thickness adopted from the ISO 11040-4 standard for the specific nominal volume as above explained. If the predetermined filling volume matches a standardized nominal volume of a standardized syringe, the standardized nominal volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this standardized or specific nominal volume. In this case, the length of the barrel may meet the length l1 indicated as indicated in Figure 1 and Table 1 of the ISO 11040-4 standard for the standardized nominal volume. If the predetermined filling volume differs from any of the nominal volumes set by the ISO 11040-4 standard, any suitable nominal volume close to the predetermined filling volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this suitable or specific nominal volume. Even if the predetermined filling volume matches a standardized nominal volume of a standardized syringe, however, any other suitable standardized nominal volume may be used as the specific nominal volume and the cylindrical barrel may be formed to meet the barrel diameters d1 and d2 and the barrel wall thickness s1 as indicated in Figure 1 and Table 1 of the ISO 11040-4 standard for this other standardized or specific nominal volume. For example, if the predetermined filling volume is 1 ml the cylindrical barrel may meet the outer diameter d1, inner diameter d2, and wall thickness s1 of a 1 ml syringe in a long version, with d1 being 8.15 mm ±0.1 mm, d2 being 6.35 mm ±0.1 mm, and s1 being approximately 0.9 mm (cf. ISO 11040-4, Tables 1 and B.1 for a nominal volume of 1 ml). In this case, the barrel length may meet the length l1 of the 1 ml syringe in the long version, being 54 mm ±0.5 mm, with the specific nominal volume being 1 ml. With the same predetermined filling volume of 1 ml, however, the cylindrical barrel may alternatively meet the outer diameter d1 (= 10.85 mm ±0.1 mm), inner diameter d2 (= 8.65 mm ±0.2 mm), and wall thickness s1 (≈ 1.1 mm) of a 1 ml syringe in a short/standard version (cf. ISO 11040-4, Tables 1 and B.1 for a nominal volume of 1 ml) with the specific nominal volume being 1 ml, or may meet the outer diameter d1 (= 6.85 mm ±0.1 mm), inner diameter d2 (= 4.65 mm ±0.1 mm), and wall thickness s1 (≈ 1.1 mm) of a 0.5 ml syringe (cf. ISO 11040-4, Tables 1 and B.1 for a nominal volume of 0.5 ml) with the specific nominal volume being 0.5 ml. In these alternative cases, the barrel length is appropriately adjusted so as to ensure that the container provides the predetermined filling volume.

Further, the above mentioned flange portion put on top of the cylindrical barrel may form, in a circumferential direction, a continuous circular flange e.g. in line with the flange of a conventional vial according to the ISO 8362-1 standard. Insofar, the flange portion may be in line with a form B of a finger flange of a standardized syringe (cf. ISO 11040-4, Figure 1, Form B). Additionally, the flange portion may be formed to comply, in terms of its cross-sectional shape, with relevant specifications of the above mentioned ISO 8362-1 standard. In particular, the flange portion may meet, in terms of its axial length/height, in terms of its upper inner edge, and/or in terms of its upper end surface, the relevant specifications as indicated in Figure 1, Figure 2, Figure 3 and Table 1 of the ISO 8362-1 standard. Specifically, the axial length/height of the flange may amount to 3.6 mm ±0.2 mm, the bevel angle of the upper inner edge may be approximately 45°, and/or the taper angle of the upper end surface of the flange may be 3° ±2° (cf. ISO 8362-1, Figures 1 to 3). Adopting the aforementioned length dimensions and angles may be useful in order to enable the flange portion of the container to cooperate with a conventional closure element as specified in the IOS 8362-2 standard. While the above mentioned 3.6 mm ±0.2 mm adopted from the ISO 8362-1 standard are preferable, the axial length/height of the flange portion may slightly differ from this standardized dimension as long as the flange portion still meets the following two functions: firstly, the flange portion shall enable handling and processing the container in conventional filling facilities and with conventional technology used for filling and processing of standardized syringes, i.e. the flange portion shall meet the function of a finger flange of a standardized syringe (cf. ISO 11040-4, Figure 1); and secondly the design of the flange portion shall allow a tight closure of the container by using an appropriate closure element, e.g. a closure element according to the ISO 8362-2 standard of a conventional standardized vial. Further, the flange portion may differ, in terms of its inner diameter, outer diameter and its lower end surface, from the relevant specifications of the ISO 8362-1 standard (cf. ISO 8362-1, Figure 1: diameter d4, diameter d2) in order to enable the flange portion to smoothly match the respective barrel dimensions. In particular, the flange portion may have a radially extending flat lower end surface unlike a standardized vial which has a tapered lower surface (cf. ISO 8362-1, Figures 1 to 3 showing a taper angle of 10° ±5°). A flat lower end surface may facilitate the handling of the container in conventional filling facilities and with conventional technology used for filling and processing of standardized syringes. Generally, however, the lower end surface of the flange portion may be formed with a taper angle as it is known from ISO 8362-1 (cf. Figures 1 to 3 showing a taper angle of 10° ±5°).

Accordingly, unlike a conventional vial the container, in particular if used as a primary packaging, according to the present invention may have, at its outer surface, no such neck constriction as it seen and specified in the ISO 8362-1 standard (cf. ISO 8362-1, Figures 1 to 3: diameter d3, height h3). On the other hand, the inner surface of the container may be finished at the upper end, i.e. the upper end section oppositely to the flange portion, in line with the finish of any appropriate one of vial models A, B, or C of the ISO 8362-1 standard (cf. ISO 8362-1, Figures 1 to 3). Specifically, the inventive flange may at least be as thick as that of a standard prefilled syringe.

The closure element is accommodated within the container in a fluid-tight contact with the circumferential wall. In other words, the closure element is held by friction or in a force-locking manner within the container. To this end, the closure element may preferably be formed so as to be elastically deformable. That is, the closure element, prior to being penetrated by the hollow needle in the process of delivery of the compounds stored in the container, fluid-tightly (i.e. compound-tightly and, notably, aseptically) seals the container by forming a leak-proof circumferential contact with its circumferential inner surface. Once the closure element has been penetrated and the discharge and penetration means, the closure element can be pushed by the penetration and discharge means against the frictional force towards the container bottom to displace and discharge the medical or pharmaceutical compound from the container into the hollow needle. Accordingly, the penetration and discharge means may serve as a piston adapted to slide or move the closure element towards the container bottom to displace and discharge the medical or pharmaceutical compound from the container into the hollow needle. That is, according to the present invention, the closure element is penetrated before it is moved towards the container bottom. Specifically, the fluid-tight contact between an outer circumference of the closure element and the inner circumferential surface of the container is such that a force greater than a penetration force for penetrating the closure element is needed to move the closure element towards the container bottom. Therefore, the closure element and the container together form a sort of slide-press-fit. Advantageously, the closure element may have a through hole aligned with the hollow needle and closed on either one side by means of a sterility diaphragm. In such a case, as a matter of course, only the diaphragm has to be penetrated. Here to penetrate means to puncture or to pierce.

The penetration and discharge unit serves to penetrate the closure element thereby to activate the inventive system, i.e. to enable the extraction and delivery of the compounds stored in the container and to finally extract the compounds. To this end, the extraction unit comprises any kind of a penetration and discharge means that has, i.e. supports, holds and/or forms, a hollow needle. By penetration of the closure element, the sterile storage of the compounds is undone at the point of penetration (and only there). Further, as above mentioned the penetration and discharge means may serve to slide or move the closure element towards the container bottom thereby to displace and discharge the medical or pharmaceutical compound from the container into the hollow needle. In this regard, the penetration and discharge means has an elongated structure with a cross-section smaller than a cross-section of the closure element and of the container, in order to enable the penetration and discharge means to plunge or enter into the container.

Further, the hollow needle may at least partially extend through a through hole formed in the penetration and discharge means. That is, the hollow needle may preferably extend to only such an extent into the penetration and discharge means that a save support is assured or may extend completely through the through hole, projecting out of it or not at the other end of the penetration and discharge means. Advantageously, the through hole in the former case is formed as a stepped hole, having a first portion accommodating the hollow needle, and a second portion having a diameter essentially equal to the inner diameter of the hollow needle, in order for a fluid channel formed within the penetration and discharge means to have an essentially constant diameter.

The inventive hollow needle serves the penetration of the closure element, and, to this end, protrudes or projects or juts out from an end face of the penetration and discharge means opposite or facing the container bottom parallel to, preferably aligned with, the longitudinal axis A. Specifically, the hollow needle is firmly held by the penetration and discharge means, preferably extending into it by a predetermined amount assuring its firm position specifically in the process of penetration. It should be emphasized that herein the term hollow needle refers to the hollow needle used to penetrate the closure element, whereas the term cannula used later refers to a hollow needle for application or administration of the compounds, that is arranged on a patients side, for example. The hollow needle may be made of metal or plastic, whereas the cannula is made of metal (standardized). The hollow needle may have at its lower end face a hole, or may have at a side of its lower end portion a hole or slit.

The link motion is defined between the first link motion portion and the second link motion portion of the extraction unit and is arranged in the extraction unit. Specifically, the first link motion portion is provided at the penetration and discharge means, while the second link motion portion is provided at the supporting element which is supported at the closure element. One of the link motion portions may be provided with one of the projection and the guiding groove, while the other of said link motion portions may accordingly be provided with the other of the projection and the guiding groove. Accordingly, as the supporting element is supported at the closure element, the extraction unit as a whole is supported at the closure element. In other words, the inventive link motion is a form-locking connection the first link motion portion forms with the second link motion portion. The form-locking connection realized by the link motion between the first link motion portion and the second link motion portion shall prevent an unintended or accidental separation of the above mentioned two parts of the extraction unit, i.e. of the penetration and discharge means and the supporting element. The inventive link motion is a deconnectable or unlockable or removable link or connection between the penetration and discharge means and the supporting element, where either one of them is provided with a slide block, i.e. a pin or peg - that is, the inventive projection -, that is moved along a predetermined trajectory by the guiding groove arranged in the respective other one of them. In particular, this implies that the inventive motion is not a screw joint.

It should be noted that the term link motion is sometimes and synonymously called motion link, both terms referring to a structure, not to a type of motion, as the first term might suggest.

Specifically, the motion of one of the first and second link motion portions as a whole with respect to the other one of the first and second link motion portions follows the guiding groove. This may be achieved if the projection as part of the one link motion portion is an element that is unflexible and steadfastly connected, preferably integrally connected, to a main body of the one link motion portion. Alternatively, the projection may in principle be flexibly connected to the main body of the one link motion portion in the way of any conventional ballpoint mechanism where a ballpoint refill is linearly movable while a projection of a compression piece thereof, usually activated by a thumb of a user to move the refill in and out, is flexible. Usually, in such a ballpoint mechanism, the compression piece is pressed once to move out the refill, and is removed in by pressing the compression piece a second time. Therefore, according to a preferred aspect of the present invention, the link motion may be designed in line with such a ballpoint mechanism.

Especially, in the latter case, the first link motion portion (i.e. the penetration and discharge means) may preferably be elastically biased relative to the second link motion portion (i.e. the supporting element) in a direction opposite to the needle penetration direction. The needle penetration direction is the moving direction of the first link motion portion, i.e. of the penetration and discharge means and of the hollow needle, with respect to the second link motion portion, i.e. the supporting element, and the closure element. An elastic force biasing the extraction unit in a direction opposite to the penetration direction may be achieved by an elastic restoring force of the closure element while penetrating the closure element. Additionally or alternatively, such an elastic force may be achieved by providing an elastic element, e.g. a compression spring, between the penetration and discharge means and one of the supporting element, container or closure element, or, in functional terms, between the first link motion portion and second link motion portion. Specifically, the elastic element may be provided between an end surface of the penetration and discharge means, which end surface faces the closure element, and the supporting element or the closure element. More specifically, the elastic element may accommodate the hollow needle projecting from the end surface of the penetration and discharge means.

The shape of the guiding groove and, therefore, the trajectory of the projection or the predetermined way, so to speak, is not specified in any way. That is, the guiding groove may generally have the shape of a regular spiral, i.e. a helical curve, or may be curved in any other way, or may be just straight, or may form spiral, curved and/or straight guiding groove sections. In other words, the guiding groove may extend (linearly) along and/or (rotationally) around the longitudinal axis so as to define a predetermined way of movement of the projection, which extends linearly along and/or rotationally around the longitudinal axis.

A straight guiding groove is the simplest form of a guiding groove and may nevertheless have advantage that the penetration and discharge means can not be retracted once the closure element has been penetrated. Therefore, the delivery system can be depolluted as a whole, without the danger of contaminating something with its former compounds.

According to an advantageous aspect of the present invention (claim 2), the penetration and discharge means is accommodated within and longitudinally guided by the supporting element.

That is, the supporting element may surround at least the first link motion portion of the penetration and discharge means. Therefore, the supporting element participates in assuring a well defined spatial relationship of the penetration and discharge means and, therefore, of the hollow needle with respect to the closure element

According to an advantageous aspect of the present invention (claim 3), the first link motion portion is inserted into the second link motion portion.

In other words, the second link motion portion may be formed as a corresponding recess or blind hole provided in the supporting element, receiving the first link motion portion projecting from the penetration and discharge means. To be specific, in a broadest sense the present invention merely requires that the link motion is formed between the first and second link motion portions. Since any link motion composed of two components implies that one of these is inserted into the respective other one, the aforementioned advantageous aspect defines the specific spatial relationship shown in Fig. 1A to 1C, for example. Alternatively, the supporting element may be formed so that the second link motion portion projects towards the penetration and discharge means and is inserted in a corresponding recess formed in the penetration and discharge means and forming the first link motion portion. In both cases, the projection or the guiding groove can be in either one of the first and second link motion portions.

According to a further advantageous aspect of the present invention (claim 4), the extraction unit further comprises a mounting element, preferably integrally, connected to the penetration and discharge means or to the supporting element, and, preferably releasably, engaged with a rim portion of the container, which rim portion defining the open second end,

Thereby, the mounting element assists in supporting and holding the extraction unit at the container unit. Advantageously, the mounting element accommodates the penetration and discharge means. That is, the mounting element advantageously may surround the penetration and discharge means and may be that component of the extraction unit that connects the extraction unit to the container unit by being connected to the rim portion. Therefore, the mounting element may participate in assuring a well defined spatial relationship of the penetration and discharge means and, therefore, the hollow needle itself with respect to the container unit and, therefore, with respect to the container proper. The rim portion of the container unit is a ring-shaped portion that comprises a circular end portion along the open second end of the container (independent of the shape of the circular end portion), and may include more than one separate elements.

According to a further advantageous aspect of the present invention (claim 5) and as above suggested, the container comprises a flange as a part of the rim portion. That is, the rim portion may be formed to have a flange.

In this case, the rim portion having the flange may be considered to form a flange portion as above mentioned and specified. It should be noted that the term flange refers to the container proper, while the term flange portion may be thought of as an extended flange and may include, besides the flange proper of the container an attachment element (cf. below where reference is made with respect to claim 6). Therefore, the flange portion can be associated with the inventive rim portion.

As noted above, the shape of the rim portion may vary. According to this advantageous aspect, the rim portion as part of the container may be a flange, i.e. a circular disc shaped protrusion extending outwardly, preferably radially outwardly, from the open second end, for example. Alternatively, the ring may be structured to have one or more steps or one or more undercuts in cross-section, for example.

According to a further advantageous aspect of the present invention (claim 6), the rim portion may comprises an attachment element fitted onto the container at the open second end, and the extraction unit is engaged with the attachment element.

That is, the attachment element may serve to connect the extraction unit to the container or mount or fit it onto it, and may, therefore, be regarded as an adapter. Therefore, the attachment element also provides a function of dimensional adaptation allowing the design to be individually (i.e. of the container as well as of the mounting element) optimized to some extent. Nevertheless, as noted above, the attachment element is regarded here as part of the container unit, specifically of its rim portion. Therefore, here, the rim portion of the container unit comprises a rim portion of the container proper together with the attachment element. Furthermore, the attachment element participates in an appropriate alignment of the extraction unit, specifically the hollow needle supporting means, with respect to the container unit it is attached to, and - together with the second link motion portion plugged into the closure element - serves as a guidance in the process of attachment of the extraction unit to the container unit and in the process of penetration of the closure element by the hollow needle.

According to a further advantageous aspect of the present invention (claim 7), the attachment element is formed as a ring-shaped cap surrounding the supporting element.

That is, the attachment element may surround the first and the second link motion portions. In other words, the supporting element and the penetration and discharge means may extend through a, preferably central, opening of the attachment element, wherein the latter, as noted above, is part of the rim portion of the container.

According to a further advantageous aspect of the present invention (claim 8), the attachment element has a first overlapping portion outwardly overlapping the rim portion, while according to a further advantageous aspect of the present invention (claim 9), the attachment element has a second overlapping portion inwardly overlapping the rim portion.

That is, in a longitudinal section including the longitudinal axis of the container, the attachment element may overlap the rim portion in a U-shape in case of inwardly and outwardly overlapping the rim portion, where the legs of the U (the I-portions thereof) may be of equal or different length.

According to a further advantageous aspect of the present invention (claim 10), the mounting element has a shape of a cup overlappingly engaged in an invertably arranged manner with respect to the container unit with the above mentioned rim portion, and the supporting element is attached to and protrudes centrally from a mounting element bottom of the mounting element and accommodates and longitudinally guides the penetration and discharge means.

Preferably, the penetration and discharge means, supporting element, the cup-shaped mounting element, and the hollow needle are essentially rotation-symmetrically arranged with respect to the longitudinal axis. Cup-shaped is to be understood as essentially equally shaped as the container, i.e. preferably right-cylindrically, preferably of circular cross-section, preferably with the mounting element bottom flat. Invertably arranged herein means that the attachment of the extraction unit to the attachment element, or generally to the rim portion, by means of the mounting element may be thought of as putting the former in an upside-down fashion with respect to the container over the latter. Therefore, centrally protruding means protruding from a central portion of the mounting element bottom into an inner space defined by the mounting element and towards the bottom of the container.

According to a further advantageous aspect of the present invention (claim 11), at least one of an edge portion of the mounting element and the rim portion is deformable in a direction orthogonal to the longitudinal axis for the extraction unit to be snappably engageable with the rim portion in a mounting process of the delivery system.

In the attached state, the ring-shaped open end portion or edge portion of the cup-shaped mounting element together with the ring-shaped attachment element - or more general: rim portion - form a circular press-fit, where, as a matter of course, the deformation is such that the former is expanded or stretched and/or the latter is compressed. The deformation state of the former and/or the latter is largest in the process of attachment, and is smallest but not zero in the final attached state, where the mounting element is snappably attached or form-lockingly connected by engagement of the cup-shaped mounting element into an undercut of the attachment element (rim portion), for example.

According to a further advantageous aspect of the present invention (claim 12), in order to be deformable, the mounting element is made of an elastic material and/or is slitted.

Therefore, the deformation mentioned above can be achieved either by an elastic formation that is based on the material only, and/or by elastic formation applied to a structure that allows portions of the mounting element to be separated by a slit, for example. Preferably, therefore, the mounting element may have a slitted structure, where one or more slits extend from the open end (edge portion) towards the mounting element bottom. Advantageously, there are formed a plurality of slits, preferably in equal intervals, along the circumference of the mounting element.

Further, the mounting element may comprise a sort of lamella structure with lamellas pointing downwards (towards the container bottom). These lamellas are flexibly pivotable about their respective fixation point to be able to circumferentially expand in the process of mounting the mounting element to the container unit.

According to a further advantageous aspect of the present invention (claim 13), the mounting element and the rim portion are provided with complementarily shaped engagement means that are engaged with each other in a snapped-in engagement state.

Advantageously, the engagement means form a hook-hook-pair, where preferably, each of the hooks is circularly shaped. The engagement means may alternatively not be complementarily shaped. For example, one of them may be a (circular) hook, while the other one may be a (circular) groove adapted to fixedly accommodate the hook, forming thereby a hook-groove-pair.

According to a further advantageous aspect of the present invention (claim 14), the penetration and discharge means has a delivery end formed as a part of a Luer lock connector.

The Luer lock connector is just one preferred (because of its standardization) inventive design of a connector connecting the inventive delivery system to another (or other) device(s) or a human body or a body of an animal. The part of a Luer lock connector that is part of the inventive delivery system may be formed as either the female or the male part thereof.

According to a further advantageous aspect of the present invention (claim 15), the penetration and discharge means has a delivery end formed as a spray valve. According to this advantageous aspect, there is no cannula. Instead, the blunt end portion of the hollow needle (the end portion opposite to the lower (in the figures) sharp end portion) advantageously transitions or blends into the spray valve, i.e. the blunt end portion of the hollow needle is formed as the spray valve. Alternatively, the spray valve is a separate component, attached to or inserted into the channel formed in or - advantageously - extending through the penetration and discharge means. Advantageously, the spray valve is recessed or set back into an upper or outer surface of the mounting element in order not to protrude therefrom.

According to a further advantageous aspect of the present invention (claim 16), the penetration and discharge means has a delivery end formed as a cannula.

A cannula is a hollow needle adapted to be plugged on a syringe to put a medical fluid into a human being or an animal, mostly intravenously. Preferably, the cannula is of a type that allows the use of the inventive system as an infusion system, for example, i.e. allows a flow of air into the container and, thereby, a flow of the compounds out of the container solely by gravitation. The term to extract used above may in this case be reasonably replaced by to empty in view of the passive draining of the compounds, i.e. the draining without the help of some kind of actuator. Preferably, the hollow needle and the cannula are integrally or one-piece formed. In such a case, the above terms refer to portions of a hollow double-needle. Preferably, the hollow needle has a tapered, bevelled or chamfered tip to allow for an easy penetration of the closure element.

According to a further advantageous aspect of the present invention (claim 17), in any of the above mentioned alternative cases, the delivery system may further comprise a protecting cap covering a delivery end of the penetration and discharge means.

The cap may be movably attachable to a rotating element, preferably removably, connected to the penetration and discharge means.

According to a further advantageous aspect of the present invention (claim 18), contact surfaces of the container bottom and the closure element are complementarily shaped, and at least one of the contact surfaces comprises a blind hole for accommodating a tip of the hollow needle.

According to this advantageous aspect, a force along the longitudinal axis is allowed to be transmitted from the penetration and discharge means to the closure element to move the closure element towards the container bottom. Complementarily shaped means herein that the opposing surfaces of the closure element and the container bottom are configured such that they are essentially in surface contact with each other when the assembly of penetration and discharge means and closure element is located at its most downward position (i.e. when the compounds initially contained in the container have been extracted as much as possible with the hollow needle in a complete extraction allowing position), where essentially means except for the blind hole (depression or recession) that is formed in one or both of them. This implies that the contact surfaces are essentially parallel to each other (except for the blind hole). That is, any convexity of one of the contact surfaces corresponds to the concave mirror image of the other one of the contact surfaces. Allowing a force along the longitudinal axis to be transmitted from the penetration and discharge means to the closure element means that the penetration and discharge means is inserted as deep as possible into the closure element, i.e. up to a position where respective end faces are in contact with each other. Up to that position, that may be called a longitudinal force transmittable position, the closure element is held by friction between its circumferential surface and in inner surface of the container and, therefore, not moved towards the container bottom but only penetrated by the hollow needle.

According to a further advantageous aspect of the present invention (claim 19), the contact surfaces are generally flat-shaped or ellipsoidically or spherically shaped.

The shapes of the contact surfaces are advantageous variations in terms of their respective manufacturing process as well as for the extraction efficiency they allow. However, in principle, the contact surfaces may be shaped arbitrarily. Preferably, the ellipsoidically or spherically shaped contract surfaces are disposed such that their respective axis of symmetry is aligned with the longitudinal axis.

According to a further advantageous aspect of the present invention (claim 20), the penetration and discharge means may be elastically biased in a direction away from the supporting element.

The direction away from the supporting element is a direction opposite to the needle penetration direction. In this regard, there may be an elastic element arranged between the penetration and discharge means and one of the supporting element, the container or the closure element (claim 21).

The needle penetration direction is the moving direction of the first link motion portion (hollow needle) with respect to all other components of the inventive delivery system, specifically with respect to the second link motion portion, when the first link motion portion is rotated in order make the hollow needle penetrate the closure element. This direction is defined herein as a forward direction. The movement of the first link motion portion in the needle penetration direction is carried out against the elastic force of the elastic element, which itself advantageously is a compression spring. As above mentioned, the elastic element may be provided between an end surface of the penetration and discharge means, which end surface faces the closure element, and the supporting element or the closure element. More specifically, the elastic element may accommodate the hollow needle projecting from the end surface of the penetration and discharge means. In this latter case, the elastic element may surround a core portion of the first link motion portion having the longitudinal axis as symmetry axis, from which the hollow needle projects. Advantageously, the first link motion portion comprises a bush portion surrounding the core portion, wherein the bush portion preferably has a flange-shaped - stopping element (flange) that is pressed against the mounting element by means of the elastic element to hold the first link motion portion in that position until it is intentionally rotated. The intentional rotation is preferably carried out by means of a rotating element manipulated by a user.

According to a further advantageous aspect of the present invention (claim 22), the supporting element comprises a tubular body coupled to the closure element, which tubular body forms the second link motion portion.

According to a further advantageous aspect of the present invention (claim 23), the penetration and discharge means comprises lamellas extending from its outer circumferential surface in a slanting upward direction and engaged with the container unit.

The lamellas are adapted to prevent a detachment of the penetration and discharge means from the rim portion. The lamellas advantageously have the shape of lateral areas of frustums or truncated cones arranged in succession or in a row along the longitudinal axis with preferably different pitch angle with respect to the longitudinal axis. The lamellas are directed slantingly upward or diagonally upward in order to be able to act as hooks preventing a detachment of the extraction unit from the attachment element, i.e. a movement of the extraction unit in the above defined retraction direction or backward direction. The term prevent means to make such a movement impossible (without damaging the system) or impeding or hampering such a movement.

According to a further advantageous aspect of the present invention (claim 24), the guiding groove extends along (i.e. linearly along) and/or around (i.e. rotationally around) the longitudinal axis.

According to a further advantageous aspect of the present invention (claim 25), the guiding groove may be such that a relative movement of the first link motion portion with respect to the second link motion portion is carried out by (i) rotating and/or advancing the first link motion portion along the longitudinal axis to longitudinally move the hollow needle by a first distance (h2) to make the hollow needle penetrate the closure element, and (ii) further rotating and/or retracting the first link motion portion to longitudinally move the hollow needle by a second distance (h2 - h1) to retract the hollow needle to a complete extraction allowing position.

Here, the complete extraction allowing position is a position that allows to extract essentially all of the compounds stored in the container to be extracted. First of all, this position is determined in that, in the penetration process, the hollow needle has to completely penetrate the closure element. Depending on the structure of the closure element, the closure element has to be over-penetrated to some extent. In the case of a diaphragm, as mentioned above, the diaphragm may be stretched and spring back. This over-penetration position is the position achieved by the first distance (h2). According to the present advantageous aspect, the hollow needle is then retracted to the complete extraction allowing position (as for terminology, the over-penetration position may be called a complete penetration position). This position is achieved by the second distance (h2 - h1, with h1 < h2). Where the closure element is fully moved towards the bottom in the process of delivery, the retraction prevents a contact of the tip of the hollow needle with the bottom and, as a consequence, a space between the closure element and the bottom. Such a space, in the case of a protruding hollow needle, would prevent the compounds included therein to be extracted. Where the closure element is not moved towards the bottom in the process of delivery (gravitation delivery, as mentioned above), the container is held in an inverted position compared to the previous variation. In such a case, the retraction of the hollow needle assures that the hollow needle does not extend above a residual amount of compounds.

According to a further advantageous aspect of the present invention (claim 26), the guiding groove may further be formed such that a relative movement of the first link motion portion with respect to the second link motion portion is further carried out by (iii) rotating the first link motion portion to a longitudinal locking position, preferably without any further longitudinal movement of the hollow needle.

The longitudinal locking position is a position in which the first link motion portion is longitudinally fixed. In the variation where the closure element is to be moved, this position may also be called a longitudinal force transmittable position, as noted above, allowing a longitudinal force, i.e. a force along the longitudinal axis sufficient to move the closure element to be applied.

Thus, in a form-locked state, the penetration and discharge means, i.e. the extraction unit, is secured to the closure element and is adapted both to piston-like push and pull the closure element. Particularly, the pulling ability allows rests of the compounds, inevitably remaining in the system after administration, to be drawn back and, therefore, to avoid spilling these rests when the extraction unit is removed from the container after having delivered the compounds

According to a further advantageous aspect of the present invention (claim 27), the delivery system may form a modular system comprising the extraction unit as a first module, and the container unit as a second module.
Advantageously, therefore, the two modules can be exchanged in case one of them is damaged or in case differently shaped containers and/or containers storing different compounds are to be used or in case one of them is to be cleaned, as long as they are correspondingly shaped to be connectable. Preferably, therefore, a user may order only a single first module used for a plurality of different compounds. In such a case, the first modules, specifically the extraction unit, may be sterilizable.

According to a further advantageous aspect of the present invention (claim 28), the second module may comprise the container, the attachment element, and the closure element as a first, a second, and a third sub-module, respectively.

Advantageously, therefore, the above division into first and second modules can be extended. This further distinction emphasizes the modular concept of the present invention.

According to a further advantageous aspect of the present invention (claim 29), at least one of said modules may be sterilizable.

Further features and advantages of the present invention will become apparent from the following detailed description of preferred embodiments with reference to the accompanying drawings. In the drawings:
Fig. 1A is a schematic drawing of a delivery system according to a preferred embodiment of the present invention in an initial state;
Fig. 1B is a schematic drawing of the delivery system of Fig. 1A in an intermediate state;
Fig. 1C is a schematic drawing of the delivery system of Fig. 1A in a final state;
Figs. 2A and 2B are schematic drawings of developed surfaces of variations of a link motion of a delivery system according to the present invention;
Fig. 3A to 3C are schematic drawings showing variations of the shape of a closure element and a container bottom of a delivery system according to the present invention;
Fig. 4 is a schematic enlarged view of an upper end of an alternative container having a flange portion;
Figs. 5A and 5B are schematic drawings illustrating an alternative of a link motion of a delivery system according to the present invention;
Figs. 5C and 5D are schematic drawings illustrating a modification of the alternative of Figs. 5A and 5B;
Fig. 6A to 6C are schematic drawings showing modifications of a delivery system according to the present invention; and
Fig. 7 is a schematic drawing showing a modification of the delivery system of the preferred embodiment shown in Fig. 1A.

Hereafter, the terms lower and upper refer to the figures.

Fig. 1A shows a schematic drawing showing an initial state of a delivery system 10 according to a preferred embodiment of the present invention.

The delivery system 10 comprises a container unit 1000 and an extraction unit 2000.

The container unit 1000 comprises a container 1100, and a closure element 1200.

The container 1100 has a hollow right-cylindrical shape, having a tube-shaped side wall 1102 and a container bottom 1104 closing the side wall 1102 at a lower first end. The side wall 1102 has a ring-shaped upper end or rim portion 1106 defining an upper and open second end and an inner surface 1108 and an outer surface 1110.

The rim portion 1106 comprises an attachment element 1114 that is a generally ring-shaped, cap-like adapter provided with a through hole 1118 and formed in such a way that it straddles the rim portion 1106 of the container 1100, wherein a ring-shaped inner portion 1120 longitudinally (along the longitudinal axis A) stretches essentially equally along the inner surface 1108 of the container 1100 towards the container bottom 1104 as a ring-shaped outer portion 1122 extends along the outer surface 1110 of the container 1100.

The inner and outer portions 1120, 1122 confine a circular groove 1124 with a circularly extending half-pipe-like roof (or bottom, when regarded upside-down compared to Fig. 1A, so that the groove forms a circular trough with walls of equal wall heights). Therefore, a lower surface 1126 of the attachment element 1114 comprises an outer ring-shaped end face 1128, an inner ring-shaped end face 1130, and a surface 1132 of the roof. The circular groove 1124 serves to accommodate a rim portion 1142 of the barrel of the container 1100. The attachment element 1114 comprises a circular detent 1134 extending outwardly and downwardly along an outer portion 1122 or the rim portion 1142, i.e. in a slanting downward direction.

The through hole 1118 has a stepped structure with a lower portion 1136 having a larger diameter than an upper portion 1138. To put it differently, the upper portion 1138 forms a ring-shaped nose extending radially inwardly.

The closure element 1200 is formed from an elastic material and accommodated within the container 1100 in a fluid-tight contact with the container to be adapted to seal the container 1100 until delivery of the compounds stored therein. To improve the sealing property, an outer surface 1202 of the closure element 1200 in contact with the inner surface 1108 of the side wall 1102 of the container 1100 has a rib structure with a plurality of circular ribs 1204. The closure element 1200 has a larger blind hole 1206 arranged centrally, i.e. symmetrically with respect to a longitudinal axis A of the container that coincides with a longitudinal axis of the inventive delivery system, and open in the direction away from the container bottom 1104. Furthermore, the closure element 1200 has a smaller blind hole 1208 also arranged centrally, but open in the direction of the container bottom 1104. The smaller blind hole 1208 is smaller than the larger blind hole 1206 both in terms of depth and in terms of width. Therefore, as shown in Fig. 1A, by the larger blind hole 1206 and the smaller blind hole 1208, there is generated a central / axial thin wall portion 1210.

The extraction unit 2000 comprises a supporting element 2150, a penetration and discharge means 2100, a mounting element 2200, and a rotating element 2300.

The penetration and discharge means 2100 comprises a hollow needle supporting means 2110. Further, as it is seen from Figs. 1A to 1C, the penetration and discharge 2100 has an elongated structure with a cross-section smaller than a cross-section of the closure element 1200 and of the container 1100, in order to fit into the container 1100.

The penetration and discharge means 2100, more specifically the hollow needle supporting means 2110 of the penetration and discharge means 2100, comprises a first link motion portion 2112, having a core portion 2114 and a bush portion 2116 integrally formed with the core portion 2114 and completely surrounding it along an upper part of its length, and a hollow needle 2122. The supporting element 2150 comprises a second link motion portion 2118 adapted to accommodate a lower part of the core portion 2114. Further, as seen in Fig. 1A an elastic element in form of a compression spring 2120 is arranged between the penetration and discharge means 2100 and the supporting element 2150. Generally, the elastic element, as already noted above, in order to provide the desired function, serves to bias the first link motion portion with respect to the second link motion portion. Therefore, the compression spring 2120 can alternatively be arranged between the lower end face 2134 of the core portion 2114 and a circular upper surface 1212 of the closure element 1200 (with a diaphragm 2140 described below arranged between them), or between a ring-shaped surface formed around the second link motion portion 2118 and the flange 2128, or between any other structures formed at the first and second link motion portion and adapted to receive the force applied by the compression spring 2120 to bias the first and second link motion portions away from each other.

The compression spring 2120 is wound around the core portion 2114 between a lower end face 2126 of the bush portion 2116 and an upper end face 2124 of the second link motion portion 2118 or of the supporting element 2150. The compression spring 2120 biases the penetration and discharge means 2100 with the first link motion portion 2112 in a direction away from the supporting element 2150 with the second link motion portion 2118, i.e. upwards or, in other words, in a direction opposite a needle penetration direction. The bush portion 2116 comprises a flange 2128 at its outer surface that is pressed in the initial state shown in Fig. 1A against the mounting element 2200, as described further below. In a surface of the lower part of the core portion 2114, there is formed a guiding groove 2130 as an engaging portion of the first link motion portion.

The second link motion portion 2118 comprises a projection 2132 formed at its inner surface. The projection 2132 is adapted to become engaged with the guiding groove 2130 to form a link motion adapted to move the penetration and discharge means 2100 relatively to the supporting element 2150 by rotating the first link motion portion 2112 relative to the second link motion portion 2118 by means of the rotating element 2300 that is torque proof fitted onto an upper end portion of the first link motion portion 2112.

The hollow needle 2122 has a lower, chamfered end portion extending downwardly beyond a lower end face 2134 of the core portion 2114, and an upper, chamfered end portion extending upwardly, beyond an upper end face 2136 of the first link motion portion 2112. That is, the hollow needle 2122 has a cannula portion extending upwardly out of the hollow needle supporting means 2110.

The supporting element 2150 has the form of a cup comprising a right-cylindrical side wall 2152 connected with its upper end face to the mounting element 2200, and a supporting element bottom 2124 at its lower end. The supporting element bottom 2124 has a central through hole 2126 through which the second link motion portion 2118 extends. Notably, an outer surface of the second link motion portion 2118 is connected, at an approximate central position in a direction of the longitudinal axis A, to a rim portion of the through hole 2126. In the preferred embodiment, the second link motion portion 2118 is formed integrally with or is integrally attached to the supporting element 2150, as seen in Fig. 1A.

The supporting element 2150 is supported at the closure element 1200 in a longitudinal direction, as seen in Fig. 1A. Specifically, the supporting element 2150 is coupled to the closure element 1200 via a lower part of the second link motion portion 2118 being accommodated in a form- and force-locking manner within the larger blind hole 1206 of the closure element 1200.

The mounting element 2200 has the form of a cup comprising a side wall 2204 and a mounting element bottom 2202 at its upper first end.

The side wall 2204 has a ring-shaped lower end portion or rim portion 2206 defining a lower and open second end of the mounting element 2200. The mounting element 2200 is invertedly and partly overlappingly arranged with respect to the container 1100. The mounting element 2200 is provided with a finger flange 2208 extending outwardly along the rim portion 2206. The mounting element 2200 is further provided with a circular detent 2210 extending inwardly and upwardly, i.e. in a slanting upward direction, along the rim portion 2206. The mounting element bottom 2202 has a circular opening through which the first link motion portion 2112 extends upwardly, and along an edge of which the flange 2128 can get in abutting contact opposing the force of the compression spring 2120.

The lower part of the second link motion portion 2118 protruding from a lower end face of the supporting element 2150 forms a needle space 2138 that downwardly extends into the larger blind hole 1206 and is aseptically sealed by the diaphragm 2140 attached to a lower, ring-shaped end face 2142 of the second link motion portion 2118. Specifically, the end face 2142 is in contact with the circular upper surface 1212 of the closure element 1200, with the diaphragm 2140 arranged there between. At a lower outer surface of the supporting element 2150, there are provided lamellas 2144, extending upwardly and outwardly away from the supporting element 2150. The lamellas 2144 are engagement with the ring-shaped nose of the attachment element 1114 to allow the penetration and discharge means 2100, specifically the supporting element 2150 to easily move downwardly with respect to attachment element 1114, but to impede a movement in the opposite direction.

The detents 1134 and 2210 form circular hooks fittingly engaging with each other in the attached initial state shown in Fig. 1A.

The rotating element 2300 is removably or non-removably fitted in a torque-proof way onto the hollow needle supporting means 2110 and comprises a central hole 2302 penetrated by the cannula portion of the hollow needle 2122. The cannula portion of the hollow needle 2122 is protected by a protecting cap 2304.

Figs. 1A to 1C show various stages or steps of the delivery of the pharmaceutical compounds stored in the container 1100.

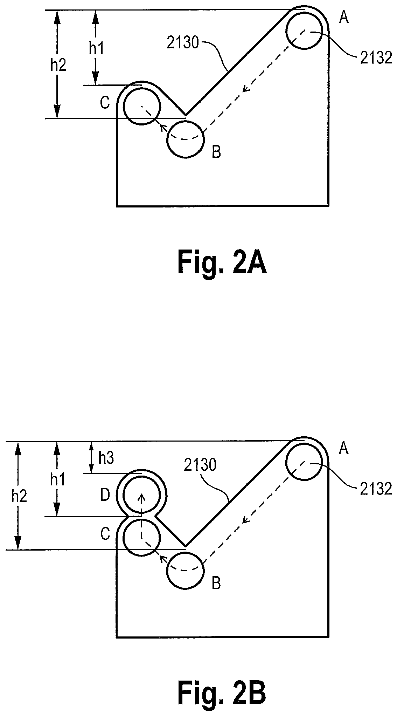

Fig. 2A shows a developed view of a preferred first variation of the link motion, formed by the projection 2132 and the guiding groove 2130. In the developed view, as noted above, the projection 2132 and the guiding groove 2130 may be part of the core portion 2114 and the second link motion portion 2118, respectively, or vice versa. That is, in Fig. 2A, the projection 2132 shown may be part of the core portion 2114 or part of the second link motion portion 2118. The same holds correspondingly for the guiding groove 2130. According to Fig. 2A, after the projection 2132 is inserted into or coupled to the guiding groove 2130 at point A, hereafter called the starting position, the projection 2132 is moved along the guiding groove 2130 by relatively rotating the core portion 2114 with respect to the second link motion portion 2118 first downwards by a longitudinal distance h2 to a position B and then upwards by a longitudinal distance h2-h1 to a position C. That is, both distances h1, h2 are measured with respect to the starting position, as shown in Fig. 2A.

Fig. 2B shows a developed view of a preferred second variation of the motion link, formed by the projection 2132 and the guiding groove 2130. The second variation differs from the first variation in that there is added a position D displaced with respect to position C radially and/or longitudinally with respect to the longitudinal axis A. Position D (> h3) is achieved by further relatively rotating the core portion 2114 with respect to the second link motion portion 2118, and is a locking position allowing a longitudinal force to be exerted on the extraction unit without return-moving the projection 2132 back to position B in the process of pushing the closure element 1200 downwards. As shown in Fig. 2B, h3<h1<h2.

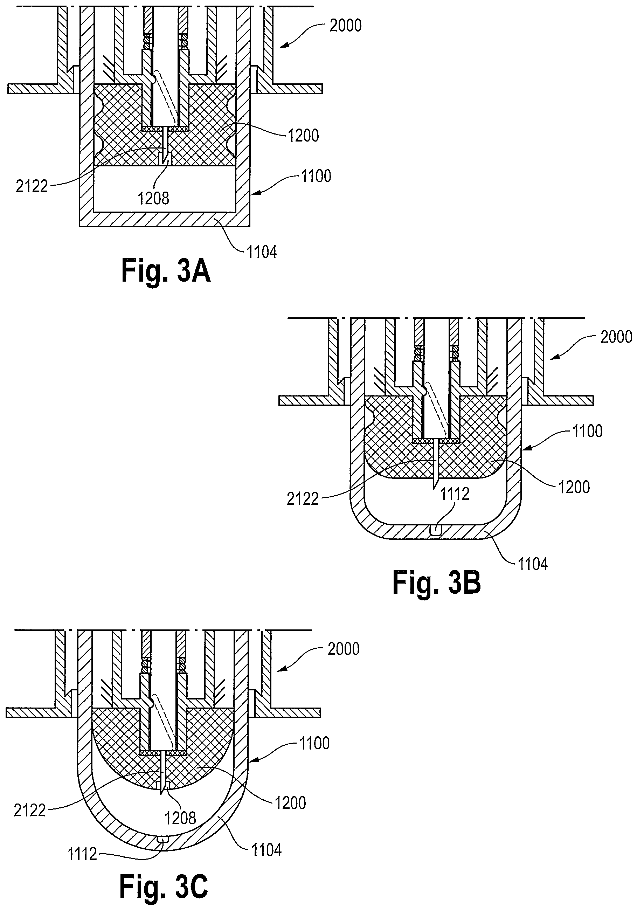

As shown in Figs. 3A to 3C, the shapes of the contact surfaces of the closure element 1200 and the container bottom 1104 correspond to each other in that any convex shape of the former is equivalent to a concave shape of the latter. That is, the contact surfaces shown are either both flat (Fig. 3A), convex (closure element 1200) and concave with flat surfaces but rounded edges (Fig. 3B), or spherical (Fig. 3C). All surface shapes aim at assuring that the rest of the compounds when the closure element 1200 is in contact with the container bottom 1104 is minimum by channeling the compounds towards the hollow needle 2122 immediately prior to the contact of the contact surfaces. Therefore, the inverse case, namely that the closure element 1200 has a concave lower surface, and the container bottom 1104 bulges convexly upwards is not reasonable. As noted above, the purpose of the smaller blind hole 1208 is, firstly, to accommodate and, therefore, to protect the hollow needle 2122 from damage and, secondly, to allow an immediate contact of the contact surfaces to reduce the amount of the compounds that can not be extracted from the container 1100 to a minimum. As shown in Fig. 3A to 3C, there are various alternatives to this end. Namely, the smaller blind hole 1208 may be provided only in either one of the closure element 1200 (Fig. 3A) and the container bottom 1104 (Fig. 3B) or in both of them (Fig. 3C). Specifically, as shown in Figs. 3B and 3C, the container bottom 1104 may be provided with a recession 1112 as well. In view of the functions of the smaller blind hole 1208 and the recession 1112, it is evident that in the case of Fig. 3C, these can be dimensioned smaller than in the cases of Figs. 3A and 3B.

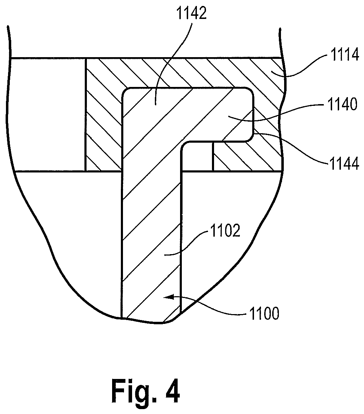

Fig. 4 shows a schematic enlarged detailed view of an alternative container 1100 having a flange 1140 extending outwardly along its rim portion 1142 (the rim portion of the container proper, i.e. the above referred to barrel). The flange 1142 is accommodated within a circular groove 1144 of an attachment element 1114', or - to put it differently - in the process of attaching the attachment element 1114' to the container 1100, the attachment element 1114' is stretched and snapped over and around the flange 1140. It should be noted that the attachment element 1114 is shown very schematically and simplified to emphasize the position of the circular groove 1144. Actually, in all other structural terms, the attachment element 1114 is identical with the attachment element 1114.

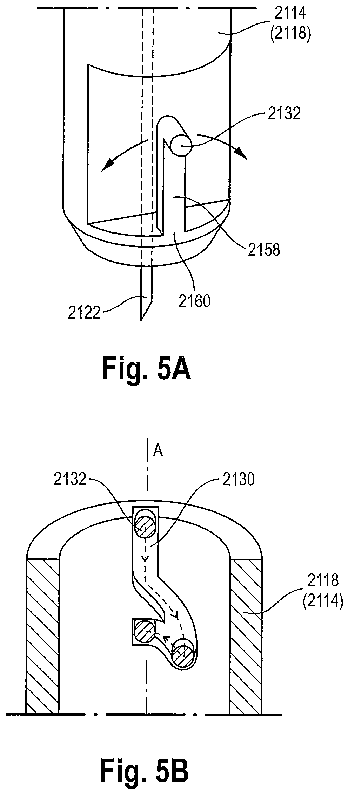

Fig. 5A and 5B are schematic drawings illustrating an alternative or variation of a link motion. In this variation, the projection 2132 may be flexibly connected to a main body of one of the first and second link motion portion - here either the core portion 2114 (the case shown in Figs. 5A, 5B) or the supporting element 2150 (shown in brackets in Figs. 5A, 5B) - by means of a flexible bar 2158, while the guiding groove 2130 is formed in the respective other one of the first and second link motion portion - here the core portion 2114 and the supporting element 2150. As shown by arrows in Fig. 5A, the flexible bar 2158 is allowed to pivot essentially circumferentially or tangentially with respect to a circumference of the main body in a plane perpendicular to the longitudinal axis about a connection point 2160. The flexible bar 2158 is integrally connected to the main body at the connection point 2160. The guiding groove 2130 may have the shape as shown in Fig. 5B, resulting in a link motion as known in principle from a ballpoint where its refill (corresponding to the penetration and discharge means 2100) is only linearily movable (here along the longitudinal axis A) while a projection of a compression piece (corresponding to the extraction unit 2000) thereof, usually activated by a thumb of a user to move the refill in and out, is flexibly suspended (corresponding to the attachment of the projection 2132 by means of the flexible bar 2158). In the present invention, as in a similar type of a link motion mechanism of a ballpoint, the extraction unit 2000 (ball point compression piece) is pressed a first time (here moved downwards) to advance and retract the hollow needle 2122 (ballpoint refill) (cf. Fig. 5B), and is pressed a second time to move in the refill.

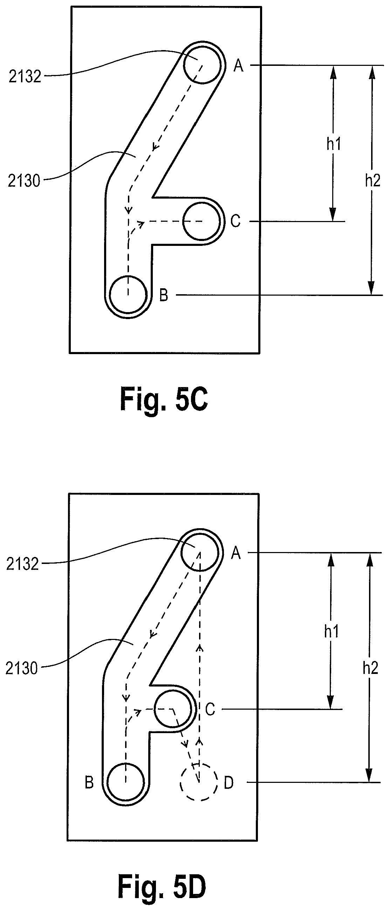

Figs. 5C and 5D are schematic drawings illustrating a modification of the alternative of the link motion of Figs. 5A and 5B. In this modification also, the link motion follows in terms of structure and functioning a link motion mechanism of a conventional ballpoint as an example. The projection 2132 is flexibly connected by means of the flexible bar 2158 (not shown in Figs. 5C and 5D) to the first or second link motion portion, while the guiding groove 2130 is formed in the other one of the first and second link motion portion. When the extraction unit 2000 is pressed a first time, the connection point 2160 of the flexible bar 2158 is moved downwards along a line slightly shifted to the right with respect to a line defined by points A and C. During that movement, the flexible bar 2158 is bent because the projection 2132 is guided within the guiding groove 2130 along a trajectory shown as dashed line from A to B to C. It should be noted that, due to the detailed structure of the mechanism (not shown here) and provided the downward movement is not too slow, the projection 2132, biased by the elastic force of the flexible bar 2158, snaps into the passage to point C only after having reached point B, i. e. in a retracting movement. By pressing the extraction unit 2000 a second time, the projection 2132 (the flexible bar 2158) snaps out of the guiding groove 2130 to allow the extraction unit 2000 to move back along said shifted line. As shown in Fig. 5D, the passage to point C may be shortened compared to that of Fig. 5C. In Fig. 5D, both the movement when the extraction unit 2000 is pressed a first time, and the movement when the extraction unit 2000 is pressed a second time, are each shown by a dashed line having in the direction of the arrows.

It should be noted that all link motions described herein exemplified in Figs. 2A, 2B and 5A to 5D are applicable to the structures of all delivery systems described with reference to the remaining figures.

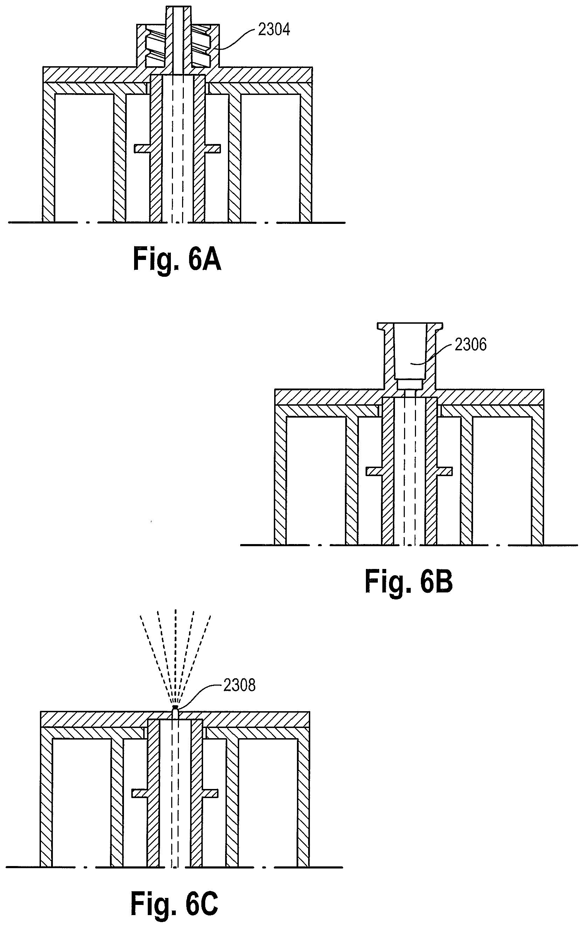

Figs. 6A to 6C show modifications corresponding to the kind of delivery of the delivery system 10. The term "kind of delivery" refers to the usage or connectivity of the inventive delivery system 10. Specifically, the hollow needle 2122 may protrude or stick out upwards from the upper end face 2136 of the core portion 2114 (or the first link motion portion 2112), as shown in Fig. 1A, in order for the delivery system 10 to be used as syringe. Alternatively, as shown in Figs. 6A and 6B, the delivery system 10 may be coupled by means of a Luer lock connection with a corresponding device, where the rotating element 2300 may be formed to serve as a Luer lock portion, for example, as shown in Figs. 6A, 6B, either as male portion 2304 as shown in Fig. 5A or a female portion 2306. The hollow needle 2122 may or may not extend up to the Luer lock portion 2304, 2306. This is indicated in Figs. 6A to 6C by the broken line that may indicate either just the through hole through the core portion 2114 or a portion of the hollow needle 2122.

As shown in Fig. 6C, the inventive delivery system 10 may alternatively be used as a spraying device having a spray valve 2308 instead of a Luer lock connection. Pursuant to the modular concept of the present invention, therefore, depending on their respective usage, differently shaped extraction units 2000 or first link motion portions 2112 may be connected to the container unit 2000. As for the hollow needle 2122, the same holds as described with respect to Figs. 6A and 6B.

Hereafter, the operation of the inventive delivery system 10 is described.

Fig. 1A shows what is referred to above as the initial state. That is, a state where the extraction unit 2000 is coupled to the container 1100 by means of the attachment element 1114 being part of the container unit 1000, but has not yet been actuated. In order to penetrate the diaphragm 2140 and the closure element 1200, the hollow needle supporting means 2110 is rotated by means of the rotating element 2300 in order to longitudinally move the hollow needle supporting means 2110, holding the hollow needle 2122, downwardly against the force of the compression spring 2120, until the lower end face of the core portion 2114 abuts onto the diaphragm 2140 and the lower tip of the hollow needle 2122 has penetrated the closure element 1200 and is located within the smaller blind hole 1208. This movement is guided by the motion link formed between guiding groove 2130 of the core portion 2114 and the projection 2132 of the second link motion portion 2118. This state is called the intermediate state, and is shown in Fig. 1B. Subsequently, the protecting cap 2310 (and optionally the rotating element 2300) is removed to expose the cannula portion of the hollow needle 2122. Finally, the mounting element 2200 is moved downwardly - advantageously by putting the fore finger and the middle finger of a hand onto the finger flange 2208 while the thumb is placed on the container bottom 1104 - while a side wall of the outer portion is passed along and outside of the side wall 1102 of the container. This downward movement takes along the side wall 2152 acting as a piston portion pushing the closure element 1200 downwardly until it gets in contact with the container bottom 1104. This state is the final state, in which essentially all components that have been stored in the container 1100 in the initial state have been delivered. The final state is shown in Fig. 1C.

It should be noted that, when the outer diameter of the rim portion 1106 due to the attachment element 1114 and/or due to the flange 1140 is larger than the outer diameter of the barrel of the container 1100, in the process Fig. 1A → Fig. 1C, an outer circumference of the rim portion 1106 glides along an inner surface of the mounting element 2200, and the mounting element 2200 (specifically its side wall 2204) is not in contact with the outer surface 1110 of the container 1100 below the rim portion 1106. Otherwise, when the outer diameter of the rim portion 1106 is not larger than the outer diameter of the barrel of the container 1100, the inner surface of the side wall 2004 glides in contact along the outer surface 1110 of the container 1100.

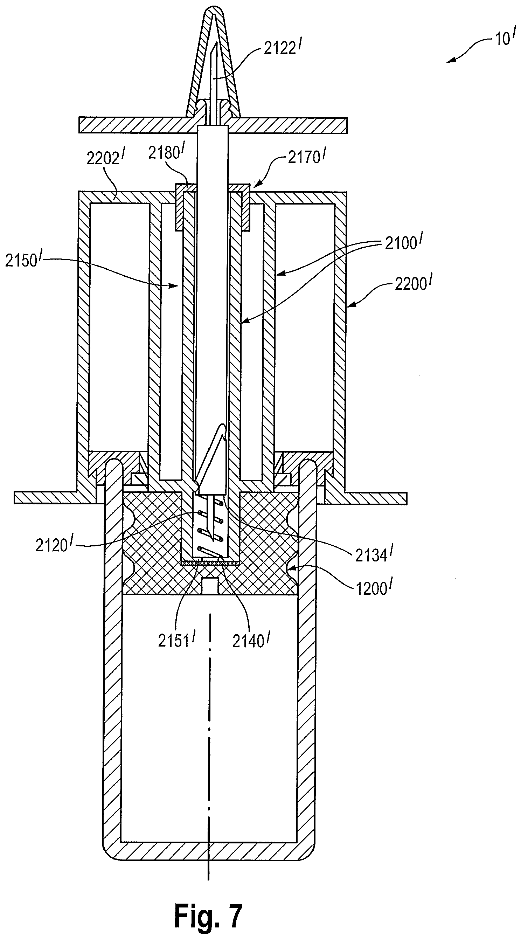

Fig. 7 schematically shows a further modification of the delivery system of the above specified preferred embodiment and any of its above mentioned modifications and variations. The delivery system shown in Fig. 7 corresponds in terms of function to the above specified preferred embodiment, so that there is no further description thereon. In terms of structure, the delivery system of Fig. 7 distinguishes from the above specified preferred embodiment only as follows. Similar to the above specified preferred embodiment, the delivery system of Fig. 7 has a compression spring 2120' arranged between the penetration and discharge means 2100' and the supporting element 2150'. However, as shown in Fig. 7 the supporting element 2150' forms at its lower end a stopper 2151' for the compression spring 2120'. Specifically, the compression spring 2120' as an elastic element is provided between a lower end surface 2134' of the penetration and discharge means 2100', which lower end surface 2134' faces the closure element 1200', and the stopper 2151' of the supporting element 2150'. More specifically, as shown in Fig. 7 the compression spring 2120' surrounds and accommodate the hollow needle 2122' projecting from the lower end surface 2134' of the penetration and discharge means 2100'. Further, as shown in Fig. 7 the supporting element 2150' abuts with its lower end which forms the stopper 2151', through the diaphragm 2140' indirectly against the closure element 1200. Further, unlike the above specified preferred embodiment, in the modified delivery system the supporting element extends in a direction opposite the needle penetration direction, i.e. in Fig. 7 upwards, until the mounting element bottom 2202'. The penetration discharge means 2100' is accommodated within the supporting element 2150' so as to be freely movable in a longitudinal direction. As seen from Fig. 7, the mounting element bottom 2202' is separated from the upper end of the supporting element 2150', so that there is an annular gap 2170' between the mounting element 2200' and the supporting element 2150'. This annular gap 2170' is closed by a cap-shaped sterility barrier 2180' surrounding the penetration and discharge means 2100'. The cap-shaped sterility barrier 2180' is formed of an elastic material, e.g. rubber polymer, and sterilely closes the annular gap 2170' by being compressed between the mounting element 2200' and the supporting element 2150'. Furthermore, the cap-shaped sterility barrier 2180' sterilely closes a radial gap between the penetration discharge means 2100' and the supporting element 2150'.

In the afore specified modification of the delivery system, the compression spring 2120' is provided between the lower end surface 2134' of the penetration and discharge means 2100' and the stopper 2151' of the supporting element 2150'. In a not shown variation the compression spring 2120' may be provided between the lower end surface 2134' of the penetration and discharge means 2100' and the closure element 1200'. In other words, the compression spring 2120' may directly abut the closure element 1200'. Of course, in this case there is no need for the supporting element 2150' to have the stopper 2151'.

### Reference Numerals

- 10: delivery system
- 1000: container unit
- 1100: container
- 1102: side wall
- 1104: container bottom
- 1106: rim portion
- 1108: inner surface
- 1110: outer surface
- 1112: recession
- 1114: attachment element
- 1118: through hole
- 1120: inner portion
- 1122: outer portion
- 1124: circular groove
- 1126: lower surface
- 1128: outer end face
- 1130: inner end face
- 1132: roof surface
- 1134: circular detent
- 1136: lower portion
- 1138: upper portion
- 1140: flange
- 1142: rim portion
- 1144: circular groove
- 1200: closure element
- 1202: outer surface
- 1204: circular ribs
- 1206: larger blind hole
- 1208: smaller blind hole
- 1210: thin wall portion
- 1212: upper surface
- 2000: extraction unit
- 2100: penetration and discharge means
- 2110: hollow needle supporting means
- 2112: first link motion portion
- 2114: core portion
- 2116: bush portion
- 2118: second link motion portion
- 2120: compression spring
- 2122: hollow needle
- 2124: upper end face
- 2126: lower end face
- 2128: flange
- 2130: guiding groove
- 2132: projection
- 2134: lower end face
- 2136: upper end face
- 2138: needle space
- 2140: diaphragm
- 2142: end face
- 2144: lamellas
- 2150: supporting element
- 2152: side wall
- 2154: supporting member bottom
- 2156: central through hole
- 2158: flexible bar
- 2160: connecting point
- 2200: mounting element
- 2202: mounting element bottom
- 2204: side wall
- 2206: rim portion
- 2208: finger flange
- 2210: circular detent
- 2300: rotating element
- 2302: central hole
- 2304: male portion of Luer lock
- 2306: female portion of Luer lock
- 2308: spray valve
- 2310: protecting cap
- A: longitudinal axis
- 1200': closure element
- 2100': penetration and discharge means
- 2120': compression spring
- 2122': hollow needle
- 2134': lower end surface
- 2140': diaphragm
- 2150': supporting element
- 2151': stopper
- 2170': annular gap
- 2180': sterility barrier
- 2200': mounting element
- 2202': mounting element bottom

## Claims

1. Delivery system (10) for delivering medical or pharmaceutical compounds, comprising:
- a container unit (1000), comprising:
- a container (1100) for storing said compounds, wherein said container has a circumferential wall (1102), a first end closed by a container bottom (1104) and an open second end (1106), and wherein said container unit (1000) has a longitudinal axis (A), and
- a closure element (1200) accommodated within said container (1100) in a fluid-tight contact with said circumferential wall (1102), and
- an extraction unit (2000) comprising a supporting element (2150) supported at the closure element (1200) and a penetration and discharge means (2100) having a hollow needle (2122) adapted to penetrate said closure element (1200),
wherein:
- said penetration and discharge means (2100) is fitted to the supporting element (2150) so as to be movable relatively to the supporting element (2150) along the longitudinal axis (A),
the delivery system being further **characterised in that**:
- said penetration and discharge means (2100) comprises a first link motion portion (2112), and said supporting element (2150) comprises a second link motion Portion (2118),
- one of said first link motion portion (2112) and said second link motion portion (2118) is provided with a projection (2132) and the respective other one is provided with a guiding groove (2130), said projection (2132) and said guiding groove (2130) being engageable to form a link motion adapted to make said penetration and discharge means (2100) move relatively to said supporting element (2150) in a predetermined way towards said container bottom (1104) thereby to make said hollow needle penetrate said closure element (1200).

2. Delivery system according to claim 1, wherein said penetration and discharge means is accommodated within and longitudinally guided by said supporting element.

3. Delivery system according to claim 1 or claim 2, wherein said first link motion portion is inserted into said second link motion portion.

4. Delivery system according to one of the preceding claims, wherein said extraction unit further comprises a mounting element, preferably integrally, connected either to said penetration and discharge means or to said supporting element, and, preferably releasably, engaged with a rim portion of said container, which rim portion defines said open second end.

5. Delivery system according to claim 4, wherein said container comprises a flange as a part of said rim portion.

6. Delivery system according to claim 4 or 5, wherein said rim portion comprises an attachment element fitted onto said container at said open second end, and said extraction unit is engaged with said attachment element.

7. Delivery system according to one of the preceding claims, wherein said attachment element is formed as a ring-shaped cap surrounding said supporting element.

8. Delivery system according to claim 7, wherein said attachment element has a first overlapping portion outwardly overlapping said container.

9. Delivery system according to claim 7 or 8, wherein said attachment element has a second overlapping portion inwardly overlapping said container.

10. Delivery system according to one of claims 4 to 9, wherein:
- said mounting element has a shape of a cup overlappingly engaged with said rim portion in an invertably arranged manner with respect to said container unit; and
- said supporting element is attached to and protrudes centrally from a mounting element bottom of said mounting element and accommodates and longitudinally guides said penetration and discharge means.

11. Delivery system according to claim 10, wherein at least one of an edge portion of said mounting element and said rim portion is deformable in a direction orthogonal to said longitudinal axis for said extraction unit to be snappably engageable with said rim portion.

12. Delivery system according to claim 11, wherein said mounting element is made of an elastic material and/or is slitted.

13. Delivery system according to one of claims 4 to 12, wherein said mounting element and said rim portion are provided with complementarily shaped engagement means that are engaged with each other in a snapped-in engagement state.

14. Delivery system according to one of the preceding claims, wherein said penetration and discharge means has a delivery end formed as a part of a Luer lock connector.

15. Delivery system according to one of claims 1 to 13, wherein said penetration and discharge means has a delivery end formed as a spray valve.

16. Delivery system according to one of claims 1 to 13, wherein said penetration and discharge means has a delivery end formed as a cannula.

17. Delivery system according to one of claims 14 to 16, further comprising a protecting cap covering a delivery end of the penetration and discharge means.

18. Delivery system according to one of the preceding claims, wherein contact surfaces of said bottom and said closure element are complementarily shaped, and at least one of said contact surfaces comprises a blind hole for accommodating a tip of said hollow needle.

19. Delivery system according to claim 18, wherein said contact surfaces are generally flat-shaped or ellipsoidically or spherically shaped.

20. Delivery system according to one of the preceding claims, wherein said first link motion portion is elastically biased in a direction away from said second link motion portion.

21. Delivery system according to claim 20, comprising an elastic element, preferably a compression spring, arranged between the penetration and discharge means and one of said supporting element, said container or said closure element.

22. Delivery system according to one of the preceding claims, wherein said supporting element comprises a tubular body coupled to the closure element, which tubular body forms the second link motion portion.

23. Delivery system according to one of the preceding claims, wherein said penetration and discharge means comprises lamellas extending from an outer circumferential surface in a slanting upward direction and engaged with the container unit.

24. Delivery system according to one of the preceding claims, wherein said guiding groove extends along and/or around said longitudinal axis.

25. Delivery system according to one of the preceding claims, wherein said guiding groove is such that a relative movement of said first link motion portion with respect to said second link motion portion is carried out by (i) rotating and/or advancing said first link motion portion along said longitudinal axis to longitudinally move said hollow needle by a first distance (h2) to make said hollow needle penetrate said closure element, and (ii) further rotating and/or retracting said first link motion portion to longitudinally move said hollow needle by a second distance (h2 - h1) to retract said hollow needle to a complete extraction allowing position.

26. Delivery system according to claim 25, wherein said guiding groove is such that said relative movement of said first link motion portion with respect to said second link motion portion is further carried out by (iii) rotating said first link motion portion to a locking position, preferably without longitudinally moving said hollow needle.

27. Delivery system according to one of the preceding claims, wherein said delivery system is a modular system comprising said extraction unit as a first module, and said container unit as a second module.

28. Delivery system according to claim 27, wherein said second module comprises said container, said attachment element, and said closure element as a first, a second, and a third sub-module, respectively.

29. Delivery system according to claim 27 or 28, wherein at least one of said modules is sterilizable.

## Patentansprüche

1. Abgabesystem (10) zum Abgeben von medizinischen oder pharmazeutischen Verbindungen, mit:
- einer Behältereinheit (1000), die umfasst:
- einen Behälter (1100) zum Speichern der Verbindungen, wobei der Behälter eine Umfangswand (1102), ein erstes Ende, das durch einen Behälterboden (1104) verschlossen ist, und ein offenes zweites Ende (1106) umfasst, und wobei die Behältereinheit (1000) eine Längsachse (A) besitzt, und
- ein Verschlusselement (1200), das in dem Behälter (1100) in einem fluiddichten Kontakt mit der Umfangswand (1102) aufgenommen ist, und
- einer Extraktionseinheit (2000) mit einem Stützelement (2150), das bei dem Verschlusselement (1200) gestützt ist, und einem Durchstech- und Abführmittel (2100), das eine Hohlnadel (2122) umfasst, die dazu geeignet ist, das Verschlusselement (1200) zu durchstechen,
wobei:
- das Durchstech- und Abführmittel (2100) an das Stützelement (2150) so angepasst ist, dass es relativ zu dem Stützelement (2150) entlang der Längsachse (A) bewegbar ist,
wobei das Abgabesystem (10) **dadurch gekennzeichnet ist, dass**:
- das Durchstech- und Abführmittel (2100) einen ersten Kulissenbewegungsabschnitt (2112) umfasst und das Stützelement (2150) einen zweiten Kulissenbewegungsabschnitt (2118) umfasst,
- einer von dem ersten Kulissenbewegungsabschnitt (2112) und dem zweiten Kulissenbewegungsabschnitt (2118) einen Vorsprung (2132) umfasst und der jeweils weitere eine Führungsnut (2130) umfasst, wobei der Vorsprung (2132) und die Führungsnut (2130) in Eingriff gebracht werden können, um so eine Kulissenbewegung zu erzeugen, die dazu geeignet ist, das Durchstech- und Abführmittel (2100) relativ zu dem Stützelement (2150) auf einem vorbestimmten Weg in Richtung des Behälterbodens (1104) zu bewegen, so dass die Hohlnadel das Verschlusselement (1200) durchsticht.

2. Abgabesystem nach Anspruch 1, wobei das Durchstech- und Abführmittel in dem Stützelement aufgenommen und durch das Stützelement längsgeführt ist.

3. Abgabesystem nach Anspruch 1 oder Anspruch 2, wobei der erste Kulissenbewegungsabschnitt in den zweiten Kulissenbewegungsabschnitt eingeführt ist.

4. Abgabesystem nach einem der vorherigen Ansprüche, wobei die Extraktionseinheit ferner ein Befestigungselement umfasst, das vorzugsweise einstückig mit entweder dem Durchstech- und Abführmittel oder dem Stützelement verbunden ist und vorzugsweise lösbar mit einem Randabschnitt des Behälters in Eingriff ist, wobei der Randabschnitt das offene zweite Ende definiert.

5. Abgabesystem nach Anspruch 4, wobei der Behälter einen Flansch als ein Teil des Randabschnitts umfasst.

6. Abgabesystem nach Anspruch 4 oder 5, wobei der Randabschnitt ein Befestigungselement umfasst, das an dem Behälter an dem offenen zweiten Ende montiert ist, und die Extraktionseinheit in Eingriff mit dem Befestigungselement ist.

7. Abgabesystem nach einem der vorherigen Ansprüche, wobei das Befestigungselement als eine ringförmige Kappe gebildet ist, die das Stützelement umgibt.

8. Abgabesystem nach Anspruch 7, wobei das Befestigungselement einen ersten Überlappungsabschnitt umfasst, der den Behälter nach außen überlappt.

9. Abgabesystem nach Anspruch 7 oder 8, wobei das Befestigungselement einen zweiten Überlappungsabschnitt umfasst, der den Behälter nach innen überlappt.

10. Abgabesystem nach einem der Ansprüche 4 bis 9, wobei:
- das Befestigungselement die Form eines Bechers hat, der sich invertierbar bezüglich der Behältereinheit überlappend in Eingriff mit dem Randabschnitt befindet; und
- das Stützelement an einem Befestigungselementeboden des Befestigungselements befestigt ist, mittig von dem Befestigungselementeboden vorragt und das Durchstech- und Abführmittel aufnimmt und längsführt.

11. Abgabesystem nach Anspruch 10, wobei wenigstens entweder ein Kantenabschnitt des Befestigungselements oder der Randabschnitt in eine Richtung senkrecht zu der Längsachse verformbar ist, so dass die Extraktionseinheit schnappbar mit dem Randabschnitt in Eingriff bringbar ist.

12. Abgabesystem nach Anspruch 11, wobei das Befestigungselement aus einem elastischen Material hergestellt und/oder geschlitzt ist.

13. Abgabesystem nach einem der Ansprüche 4 bis 12, wobei das Befestigungselement und der Randabschnitt komplementär geformte Eingriffsmittel umfassen, die in einem eingeschnappten Eingriffszustand miteinander in Eingriff sind.

14. Abgabesystem nach einem der vorherigen Ansprüche, wobei das Durchstech- und Abführmittel ein Abgabe-Ende umfasst, das als Teil eines Luer-Lock-Verbinders ausgebildet ist.

15. Abgabesystem nach einem der Ansprüche 1 bis 13, wobei das Durchstech- und Abführmittel ein Abgabe-Ende umfasst, das als Sprühventil ausgebildet ist.

16. Abgabesystem nach einem der Ansprüche 1 bis 13, wobei das Durchstech- und Abführmittel ein Abgabe-Ende umfasst, das als Kanüle ausgebildet ist.

17. Abgabesystem nach einem der Ansprüche 14 bis 16, das ferner eine Schutzkappe umfasst, die ein Abgabe-Ende des Durchstech- und Abführmittels überdeckt.

18. Abgabesystem nach einem der vorherigen Ansprüche, wobei Kontaktoberflächen des Bodens und des Verschlusselements komplementär geformt sind und wenigstens eine der Kontaktoberfläche ein Blindloch zur Aufnahme einer Spitze der Hohlnadel umfasst.

19. Abgabesystem nach Anspruch 18, wobei die Kontaktoberflächen allgemein flach oder ellipsoid oder sphärisch geformt sind.

20. Abgabesystem nach einem der vorherigen Ansprüche, wobei der erste Kulissenbewegungsabschnitt in eine Richtung von dem zweiten Kulissenbewegungsabschnitt weg elastisch vorgespannt ist.

21. Abgabesystem nach Anspruch 20, das ein elastisches Element umfasst, vorzugsweise eine Druckfeder, das zwischen dem Durchstech- und Abführmittel und entweder dem Stützelement, dem Behälter oder dem Verschlusselement angeordnet ist.

22. Abgabesystem nach einem der vorherigen Ansprüche, wobei das Stützelement einen röhrenförmigen Körper umfasst, der mit dem Verschlusselement verbunden ist, wobei der röhrenförmige Körper den zweiten Kulissenbewegungsabschnitt bildet.

23. Abgabesystem nach einem der vorherigen Ansprüche, wobei das Durchstech- und Abführmittel Lamellen umfasst, die sich von einer äußeren Umfangsoberfläche in eine Richtung schräg nach oben erstrecken und in Eingriff mit der Behältereinheit befinden.

24. Abgabesystem nach einem der vorherigen Ansprüche, wobei sich die Führungsnut entlang und/oder um die Längsachse erstreckt.

25. Abgabesystem nach einem der vorherigen Ansprüche, wobei die Führungsnut derart ist, dass eine Relativbewegung des ersten Kulissenbewegungsabschnitts bezüglich des zweiten Kulissenbewegungsabschnitts durch (i) Drehen und/oder Vorwärtsbewegen des ersten Kulissenbewegungsabschnitts entlang der Längsachse zum Längsbewegen der Hohlnadel um eine erste Distanz (h2), so dass die Hohlnadel das Verschlusselement durchsticht, und (ii) Weiterdrehen und/oder Zurückziehen des ersten Kulissenbewegungsabschnitts zum Längsbewegen der Hohlnadel um eine zweite Distanz (h2 - h1), so dass die Hohlnadel zu einer Position, die ein vollständiges Extrahieren erlaubt, zurückgezogen wird, durchgeführt wird.

26. Abgabesystem nach Anspruch 25, wobei die Führungsnut derart ist, dass die Relativbewegung des ersten Kulissenbewegungsabschnitts bezüglich des zweiten Kulissenbewegungsabschnitts durch (iii) Drehen des ersten Kulissenbewegungsabschnitts zu einer Verriegelungsposition, vorzugsweise ohne Längsbewegung der Hohlnadel, fortgesetzt wird.

27. Abgabesystem nach einem der vorherigen Ansprüche, wobei das Abgabesystem ein modulares System ist, das die Extraktionseinheit als ein erstes Modul und die Behältereinheit als ein zweites Modul umfasst.

28. Abgabesystem nach Anspruch 27, wobei das zweite Modul den Behälter, das Befestigungselement und das Verschlusselement als ein erstes, ein zweites bzw. ein drittes Teilmodul umfasst.

29. Abgabesystem nach Anspruch 27 oder 28, wobei wenigstens eines der Module sterilisiert ist.

## Revendications

1. Système d'administration (10) pour administrer des composés médicaux ou pharmaceutiques, comprenant :
- une unité de récipient (1000) comprenant :
-- un récipient (1100) pour stocker lesdits composés, dans lequel ledit récipient a une paroi circonférentielle (1102), une première extrémité fermée par un fond de récipient (1104) et une seconde extrémité ouverte (1106), et dans lequel ladite unité de récipient (1000) a un axe longitudinal (A), et
-- un élément de fermeture (1200) logé à l'intérieur dudit récipient (1100) en contact étanche aux fluides avec ladite paroi circonférentielle (1102), et
- une unité d'extraction (2000) comprenant un élément support (2150) supporté au niveau de l'élément de fermeture (1200) et un moyen de pénétration et d'administration (2100) ayant une aiguille creuse (2122) adaptée pour pénétrer dans ledit élément de fermeture (1200),
dans lequel :
- ledit moyen de pénétration et d'administration (2100) est monté sur l'élément support (2150) de manière à pouvoir se déplacer par rapport à l'élément support (2150) le long de l'axe longitudinal (A),
le système d'administration étant en outre **caractérisé en ce que** :
- ledit moyen de pénétration et d'administration (2100) comprend une première portion de commande à coulisse (2112) et ledit élément support (2150) comprend une seconde portion de commande à coulisse (2118),
- l'une parmi ladite première portion de commande à coulisse (2112) et ladite seconde portion de commande à coulisse (2118) est dotée d'une saillie (2132) et l'autre respective est dotée d'une rainure de guidage (2130), ladite saillie (2132) et ladite rainure de guidage (2130) pouvant venir en prise pour former une commande à coulisse adaptée pour amener ledit moyen de pénétration et d'administration (2100) à se déplacer par rapport audit élément support (2150) d'une façon prédéterminée en direction dudit fond de récipient (1104) pour ainsi amener ladite aiguille creuse à pénétrer dans ledit élément de fermeture (1200).

2. Système d'administration selon la revendication 1, dans lequel ledit moyen de pénétration et d'administration est logé à l'intérieur et guidé longitudinalement par ledit élément support.

3. Système d'administration selon la revendication 1 ou la revendication 2, dans lequel ladite première portion de commande à coulisse est insérée à l'intérieur de ladite seconde portion de commande à coulisse.

4. Système d'administration selon l'une des revendications précédentes, dans lequel ladite unité d'extraction comprend en outre un élément de montage relié, de préférence d'un seul tenant, soit audit moyen de pénétration et d'administration, soit audit élément support, et en prise, de préférence de manière amovible, avec une portion de rebord dudit récipient, laquelle portion de rebord définit ladite seconde extrémité ouverte.

5. Système d'administration selon la revendication 4, dans lequel ledit récipient comprend une bride en tant que partie de ladite portion de rebord.

6. Système d'administration selon la revendication 4 ou 5, dans lequel ladite portion de rebord comprend un élément de fixation monté sur ledit récipient à ladite seconde extrémité ouverte, et ladite unité d'extraction est en prise avec ledit élément de fixation.

7. Système d'administration selon l'une des revendications précédentes, dans lequel ledit élément de fixation se présente sous forme de capuchon en forme d'anneau entourant ledit élément support.

8. Système d'administration selon la revendication 7, dans lequel ledit élément de fixation a une première portion chevauchante, chevauchant vers l'extérieur ledit récipient.

9. Système d'administration selon la revendication 7 ou 8, dans lequel ledit élément de fixation a une seconde portion chevauchante, chevauchant vers l'intérieur ledit récipient.

10. Système d'administration selon l'une des revendications 4 à 9, dans lequel :
- ledit élément de montage a la forme d'une coupe en prise de manière chevauchante avec ladite portion de rebord d'une manière agencée par inversion par rapport à ladite unité de récipient ; et
- ledit élément support est fixé à et fait saillie au centre à partir d'un fond d'élément de montage dudit élément de montage et loge et guide longitudinalement ledit moyen de pénétration et d'administration.

11. Système d'administration selon la revendication 10, dans lequel au moins l'une parmi une portion de bord dudit élément de montage et ladite portion de rebord peut se déformer dans une direction orthogonale audit axe longitudinal pour que ladite unité d'extraction puisse venir en prise par encliquetage avec ladite portion de rebord.

12. Système d'administration selon la revendication 11, dans lequel ledit élément de montage est fait d'un matériau élastique et/ou est fendu.

13. Système d'administration selon l'une des revendications 4 à 12, dans lequel ledit élément de montage et ladite portion de rebord sont dotés de moyens de mise en prise de formes complémentaires qui sont en prise l'un avec l'autre dans un état de prise par encliquetage.

14. Système d'administration selon l'une des revendications précédentes, dans lequel ledit moyen de pénétration et d'administration a une extrémité d'administration formée en tant que partie d'un raccord Luer Lock.

15. Système d'administration selon l'une des revendications 1 à 13, dans lequel ledit moyen de pénétration et d'administration a une extrémité d'administration se présentant sous forme de soupape de pulvérisation.

16. Système d'administration selon l'une des revendications 1 à 13, dans lequel ledit moyen de pénétration et d'administration a une extrémité d'administration se présentant sous forme de canule.

17. Système d'administration selon l'une des revendications 14 à 16, comprenant en outre un capuchon de protection recouvrant une extrémité d'administration du moyen de pénétration et d'administration.

18. Système d'administration selon l'une des revendications précédentes, dans lequel des surfaces de contact dudit fond et dudit élément de fermeture sont de formes complémentaires, et au moins l'une desdites surfaces de contact comprend un trou borgne pour loger une pointe de ladite aiguille creuse.

19. Système d'administration selon la revendication 18, dans lequel lesdites surfaces de contact sont généralement de forme plate ou de forme ellipsoïdale ou sphérique.

20. Système d'administration selon l'une des revendications précédentes, dans lequel ladite première portion de commande à coulisse est sollicitée élastiquement dans une direction s'éloignant de ladite seconde portion de commande à coulisse.

21. Système d'administration selon la revendication 20, comprenant un élément élastique, de préférence un ressort de compression, agencé entre le moyen de pénétration et d'administration et l'un parmi ledit élément support, ledit récipient ou ledit élément de fermeture.

22. Système d'administration selon l'une des revendications précédentes, dans lequel ledit élément support comprend un corps tubulaire couplé à l'élément de fermeture, dont le corps tubulaire forme la seconde portion de commande à coulisse.

23. Système d'administration selon l'une des revendications précédentes, dans lequel ledit moyen de pénétration et d'administration comprend des lamelles s'étendant depuis une surface circonférentielle externe dans une direction inclinée vers le haut et en prise avec l'unité de récipient.

24. Système d'administration selon l'une des revendications précédentes, dans lequel ladite rainure de guidage s'étend le long et/ou autour dudit axe longitudinal.

25. Système d'administration selon l'une des revendications précédentes, dans lequel ladite rainure de guidage est telle qu'un déplacement relatif de ladite première portion de commande à coulisse par rapport à ladite seconde portion de commande à coulisse est réalisé en (i) faisant tourner et/ou avancer ladite première portion de commande à coulisse le long dudit axe longitudinal pour déplacer longitudinalement ladite aiguille creuse d'une première distance (h2) pour amener ladite aiguille creuse à pénétrer dans ledit élément de fermeture, et (ii) faisant davantage tourner et/ou rétractant ladite première portion de commande à coulisse pour déplacer longitudinalement ladite aiguille creuse d'une seconde distance (h2 - h1) pour rétracter ladite aiguille creuse vers une position permettant une extraction complète.

26. Système d'administration selon la revendication 25, dans lequel ladite rainure de guidage est telle que ledit déplacement relatif de ladite première portion de commande à coulisse par rapport à ladite seconde portion de commande à coulisse est en outre réalisé en (iii) faisant tourner ladite première portion de commande à coulisse vers une position de verrouillage, de préférence sans déplacer longitudinalement ladite aiguille creuse.

27. Système d'administration selon l'une des revendications précédentes, dans lequel ledit système d'administration est un système modulaire comprenant ladite unité d'extraction en tant que premier module, et ladite unité de récipient en tant que second module.

28. Système d'administration selon la revendication 27, dans lequel ledit second module comprend ledit récipient, ledit élément de fixation, et ledit élément de fermeture en tant que premier, deuxième et troisième sous-modules, respectivement.

29. Système d'administration selon la revendication 27 ou 28, dans lequel au moins l'un desdits modules est stérilisable.
